**Europäisches Patentamt**

**European Patent Office**

**Office européen des brevets**

(19)

(11) **EP 1 253 423 A2**

(12) **EUROPEAN PATENT APPLICATION**

(43) Date of publication:
**30.10.2002 Bulletin 2002/44**

(51) Int Cl.$^7$: **G01N 33/12**, G01N 27/02

(21) Application number: **02380091.5**

(22) Date of filing: **26.04.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.04.2001 ES 200100990**

(71) Applicant: **NTE, S.A.**
**08186 Lliça d'Amunt (Barcelona) (ES)**

(72) Inventors:
• **Elvira Canas, Jordi**
**08014 Barcelona (ES)**

• **Riu Costa, Pere**
**08034 Barcelona (ES)**
• **Rosell Ferrer, Xavier**
**08034 Barcelona (ES)**
• **Bragos Bardia, Ramon**
**08034 Barcelona (ES)**

(74) Representative: **Davila Baz, Angel et al**
**c/o Clarke, Modet & Co.,**
**Goya, 11**
**28001 Madrid (ES)**

(54) **Method and apparatus for obtaining physical and/or chemical characteristics of a biological medium**

(57)     A method of obtaining physical and/or chemical characteristics of a biological medium, comprising submitting said medium to at least two currents I. of different strength and frequency $f_I$, by means of a first group of electrodes, measuring the voltage drop $U_i$ in said medium by means of a second group of electrodes, positioning a first element of the first group of electrodes and a second element of the second group of electrodes in a first zone of said medium, and positioning a third element of the first group of electrodes and a fourth element of the second group of electrodes in a second zone of said medium. Values of electrical impedance $Z_i$ are calculated at the, at least, two frequencies $f_i$ applied, and are adjusted to the curve:

$$Z(f) = R_\infty + \frac{R_0 + R_\infty}{I + (j\frac{f}{f_c}I - \alpha}$$

where $R_0$ and $R_\infty$ are the resistances exhibited by said medium in direct current and at very high frequency, respectively; $f_c$ is the frequency at which the maximum of the imaginary part is obtained; and $\alpha$ is a parameter connected with the type of tissue of the biological medium being measured.

FIG. 2

**EP 1 253 423 A2**

## Description

FIELD OF THE INVENTION

The present invention relates to a method and apparatus for obtaining physical and/or chemical characteristics of a biological medium, for example meat. The physical and/or chemical characteristics obtained serve for assessing the quality of said meat, or in the case of ground meat, for determining its composition.

This invention presents a portable apparatus that measures the final quality of meat on its arrival at the factory and an apparatus that can be connected to mixers and mincers and determines the composition of ground meat.

BACKGROUND TO THE INVENTION

Undertakings in the meat industry employ methods for assessing the final quality of meat on arrival of the raw material at the factory. It would be desirable to be able to estimate, at the time of arrival at the factory, the final quality that the meat will have once it has been processed. For example, if the raw material is a leg of pork, a company that produces cured ham would be interested in estimating the quality of the ham obtained at the end of the process, but by taking measurements when the leg of pork arrives at the factory. Obviously, this would enable the company to reject, on arrival at the factory, meat that will not have the desired final quality.

Furthermore, these companies are also interested in determination of the composition of ground meat during the process of production of certain meat products (sausages, hamburgers, etc.). Mincers and mixers of large volume (with capacity from 200 to 600 kg) are used during these processes. It is necessary to measure the composition of the meat leaving the mincers and of the meat that is blended in the mixers, so as to be able to make corrections to the composition during the production process.

Spanish patent application P9300378, of the same applicant, relates to a method of determining the composition of substances of the meat type using the method of electrical impedance spectroscopy. The method comprises stages of applying, to the meat product to be analyzed, at least two currents of different strength and selectable frequencies, by means of two electrodes, then determining the voltage drop of each current applied, to obtain a parameter represented by the ratio of the impedances of the applied currents, found from the measured values of the voltage drops. The parameter obtained is independent of the position and degree of contact of the electrodes.

On the other hand, Spanish patent application P9601238, of the same applicant, relates to apparatus for monitoring the technological quality of meat. The apparatus comprises a microprocessor, which generates a square-wave signal; a voltage/current converter; a calibration circuit and a circuit for measuring electrical conductivity, made up of a pair of collector electrodes and a pair for current application. The circuits can be connected selectively to the output current of the converter. The apparatus is completed with an instrumentation amplifier, a demodulator and an analogue/digital converter.

DESCRIPTION OF THE INVENTION

The aim of the present invention is to solve the problems described by means of a method of obtaining physical and/or chemical characteristics of a biological medium. Said method comprises submitting said biological medium to at least two currents of different strength and frequency, via a first group of electrodes, then measuring, by means of a second group of electrodes, the voltage drop in said biological medium; to this end, a first element of said first group of electrodes and a second element of said second group of electrodes are positioned in a first zone of said biological medium, and a third element of said first group of electrodes and a fourth element of said second group of electrodes are positioned in a second zone of said biological medium.

The values of the applied current and measured voltage are used for calculating values of electrical impedance at the, at least, two frequencies applied.

The frequencies can be selectable, said selection depending on the type of biological medium being measured.

The measurement of electrical impedance can be corrected with the measurement of temperature and conductivity according to known linear coefficients.

The calculated values of electrical impedance are adjusted to the following curve:

$$[1] \qquad Z(f) = R_\infty + \frac{R_0 - R_\infty}{1 + (j\frac{f}{f_c})^{1-\alpha}}$$

where $R_0$ and $R_\infty$ are the resistances exhibited by the biological medium in direct current and at very high frequency, respectively; $f_c$ is the frequency at which the maximum of the imaginary part is obtained; and $\alpha$ is a parameter connected with the type of tissue of the biological medium being measured.

Preferably, the biological medium is submitted to three currents of different strength and frequency. The frequencies can be selectable, and selection depends on the type of biological medium to be measured. Typically, the three frequencies are: a low frequency close to $R_0$, a frequency that is sufficiently high, close to $R_\infty$, and a frequency close to $f_c$.

The method of the invention is preferably applied for determining physical and/or chemical characteristics of unprocessed meat on arrival at the factory, for estimating the quality of said meat once processed.

The ratio of the impedance at high frequency to the impedance at low frequency is proportional to the ratio of the extracellular volume to the total volume (sum of intracellular and extracellular volumes) of the biological medium being measured. This ratio is of the utmost importance in meat processing involving meat curing or cooking. The greater the extracellular volume (or unbound volume), the easier the meat will lose water. Conversely, if the intracellular volume is high, the loss of water in processes of curing or cooking will be less, compared with other meat with lower intracellular volume.

The intramuscular index I2 of the unprocessed meat is found from the expression:

$$[2] \qquad I2 = C \cdot Weight \cdot q \cdot f(R_\infty)$$

where C is an adjustment constant; Weight is the weight of said unprocessed meat; q is a geometric parameter of the meat being measured, and $f(R_\infty)$ is a function of the resistance at infinite frequency, calculated according to the model or measured at a sufficiently high frequency.

The method of the present invention is also applied preferably to the determination of physical and/or chemical characteristics of ground meat for determining the composition of said ground meat.

In the case of ground meat, the fat content FC is found from the expression:

$$[6] \qquad FC = B + D \cdot f(R_0)$$

where B and D are adjustment constants of the linear model when comparing the FC measured by an alternative method and the term $f(R_0)$, and $f(R_0)$ is a function of the resistance at low frequency.

A special case of this expression would be a potential function $FC = 1 - D \cdot (R_0)^a$, where "a" is a constant.

Measurement of electrical impedance at high frequency provides information on the water content of ground meat; thus, the total water content TWC is found from the expression:

$$[7] \qquad TWC = ko_w + k_w \cdot G^2 \cdot f(R_\infty)$$

where $ko_w$ and $k_w$ are adjustment constants of the linear model when comparing the TWC measured by an alternative method with the term $G^2 \cdot f(R_\infty)$; G is a geometric term of a container where the sample of ground meat is being measured.

A special case of this expression would be a relation that is inversely proportional to TWC:

$$TWC = ko_w + \frac{k_w \cdot G^2}{[R_\infty]}$$

The protein content PC of the measured sample is found by subtracting, from the weight of the sample, its total water content, and the content of fat and ash, according to the expression:

$$[8] \qquad PC = Weight - TWC - FC - E$$

where Weight is the weight of the sample of ground meat; TWC is the total water content found using expression [7]; FC is the fat content found from expression [6], and E is obtained from standard tables or by calibration.

The first and second zones for locating the electrodes on the sample of meat to be measured are the Pectineus muscle and the *Biceps femoris* muscle, or the *Semimembranosus* muscle in its distal part and the *Biceps femoris* muscle or the *Semimembranosus* muscle in its distal part and the *Semimembranosus* muscle in its proximal part.

The invention also relates to apparatus for obtaining physical and/or chemical characteristics of a biological medium, comprising a microprocessor, a keyboard, a screen or monitor, power supply for said microprocessor, a first group of electrodes for current injection consisting of at least one first and one third element and a second group of electrodes for measuring the voltage drop, consisting of at least one second and one fourth element; in addition the apparatus includes a differential amplifier, a demodulator and a calibration network.

The apparatus contains, at least, two filters, which give rise to at least two signals of different frequency that are applied alternately by means of a multiplexer to the first group of electrodes. Preferably, the apparatus comprises three filters, so as to generate three signals at three different frequencies.

In the case when the biological medium to be measured is ground meat, the apparatus includes means for connecting to a system for controlling a mixer of ground meat, said control system sending a signal to the microprocessor each time some blades of the mixer pass over the first and second group of electrodes.

Said control system of the mixer or mincer of ground meat is typically a PLC.

The first group of electrodes and the second group of electrodes can be mounted on the same first support and the first element is separated from the second element and the third element from the fourth element by a group of insulating elements.

The first element and the second element can be mounted on a first support and the third element and the fourth element are mounted on a second support.

In addition, the first group of electrodes includes at least one fifth element and the second group of electrodes includes at least one sixth element, all the elements being mounted on the same first support.

In any one of the cases, the apparatus includes at least one surface temperature probe that is located on the first support.

Preferably, the apparatus includes a deep temperature probe, which is located on one of the insulating elements.

BRIEF DESCRIPTION OF THE DRAWINGS

There now follows a very brief description of a number of drawings that will aid better understanding of the invention and which relate expressly to one embodiment of said invention, which is described as a non-limiting example thereof.

Fig. 1 is an equivalent circuit of what is measured using the method of the invention.
Fig. 2 shows a first embodiment of the first and second group of electrodes, for the case of unprocessed meat.
Fig. 3 shows a second embodiment of the first and second group of electrodes, for the case of unprocessed meat.
Fig. 4 shows a third embodiment of the first and second group of electrodes, for the case of ground meat.
Figs. 5-7 show possible distributions of the first zone and second zone where the first and second groups of electrodes are located.
Fig. 8 shows a block diagram of a first embodiment of the apparatus of the invention.
Fig. 9 shows a block diagram of a second embodiment of the apparatus of the invention.

DESCRIPTION OF A PREFERRED EMBODIMENT OF THE INVENTION

The invention relates to a method and apparatus (1, 1') for obtaining physical and/or chemical characteristics of the biological medium that is being measured. The apparatus measures the quality of said biological medium for example meat, using the method of electrical impedance spectroscopy.

Electrical impedance spectroscopy consists of measuring the electrical impedance at various frequencies ($f_i$). The measurement process begins, according to a preferred embodiment of the invention, with generation of electric current digitally, although it can also be generated by analogue means, and its application to the biological medium (10) to be measured (for example meat) via a first group of electrodes (20, 20'). The voltage drop in the biological medium (10) is measured by means of another, second group of electrodes (30, 30'). This is a four-wire measurement, which largely makes the result of the measurements of impedance independent of the contact of the electrodes with the medium.

The ratio of the voltage signal $U_i$ to the electric current signal $I_i$ is the electrical impedance $Z_i$. The process is repeated for various frequencies $f_i$ from 1 kHz to 1 MHz in the case of measurements on meat. The frequencies can be selectable and said selection depends on the type of meat. The electrical impedance can be corrected with the measurement of temperature and conductivity according to known linear coefficients.

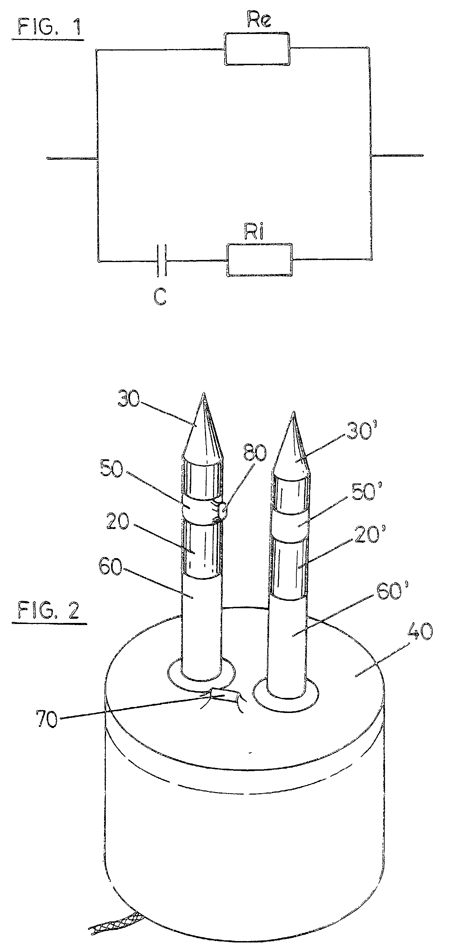

An equivalent circuit of what is measured in a biological medium by this method is shown in Fig. 1. When the frequency of the applied current is very high, the capacitor C behaves as if it were short-circuited and all the current flows through the intracellular medium with an electrical resistance Ri and through the extracellular medium with a resistance Re. When the frequency is very low, capacitor C behaves as if it were in open circuit and forces all the current to pass through the extracellular medium only.

Fig. 2 shows a first example of embodiment of the first and second group of electrodes, for the case when the biological medium (10) is unprocessed meat. In accordance with the embodiment shown in Fig. 2, a first element (20) of the first group of electrodes (20, 20') together with a second element (30) of the second group of electrodes (30, 30'), and a third element (20') of the first group of electrodes (20, 20') together with a fourth element (30') of the second group of electrodes (30, 30') are mounted on a first support (40), and are separated from one another by a group of insulating elements (50, 50').

In accordance with this embodiment, the insulating elements (50, 50') are coverings of insulating material, of the washer type. These insulating elements (50, 50') prevent electrical contact between the electrodes.

The first group of electrodes (20, 20') and the second group of electrodes (30, 30') are introduced into the biological medium (10) (for example in the semimembranous muscle of a leg of pork, in the *Longissimus dorsi* of dressed pork, in the *Biceps femoris* of a leg of pork, etc.) as far as the limit presented by the first support (40).

According to this embodiment shown in Fig. 2, the first group of electrodes (20, 20') has a covering of insulating material (60, 60'), enabling effective measurement of the electrical impedance to be taken in the interior of the region of the biological medium (10) being measured. In addition, a surface temperature probe (70) is incorporated, located on the first support (40), which measures the surface temperature of the leg of meat when the electrodes are inserted to the limit. A deep temperature probe (80), i.e. for measuring the temperature inside the region where the electrical impedance is being measured, is also incorporated. The groups of electrodes in Fig. 2 make it possible to effect local measurement of a meat sample.

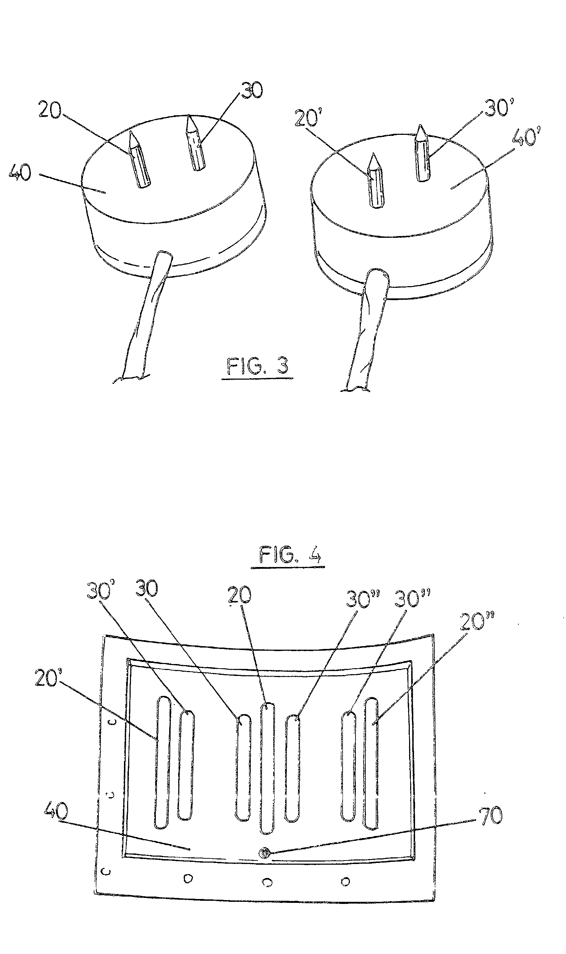

Fig. 3 shows a second embodiment of the first and second group of electrodes, for the case of unprocessed meat. This second embodiment of the electrodes is intended for measuring larger regions and can include various parts of the meat sample in the measurement. Concretely, they are thinner electrodes, and they produce a hole of smaller diameter than that produced by the electrodes in Fig. 2.

In accordance with the embodiment shown in said Fig. 3, a first element (20) of the first group of electrodes (20, 20') together with a second element (30) of the second group of electrodes (30, 30') are mounted on a first support (40), and a third element (20') of the first group of electrodes (20, 20') together with a fourth element (30') of the second group of electrodes (30, 30') are mounted on a second support (40'). In this way, on each support there will be one electrode for current injection and another one for detecting the voltage drop. Said first support (40) will be located in a first zone of the meat sample, and the second support (40') will be located on a second zone, in order to measure different regions. These groups can also have temperature probes (not shown for this embodiment).

Fig. 4 shows a third embodiment of the first and second group of electrodes, for the case of ground meat.

In this case, the first group of electrodes includes, in addition to the first element (20) and the third element (20'), a fifth element (20''); similarly, the second group of electrodes includes, in addition to the second element (30) and the fourth element (30'), two sixth elements (30''). In this way, a double measurement of voltage is achieved via the second element (30) and the fourth element (30') and between the two sixth elements (30'').

In this embodiment, all the elements (20, 20', 20'', 30, 30', 30'') are mounted on the same first support 40), which consists of a surface of insulating material in which said elements are embedded, the whole giving a smooth, continuous surface. This surface replaces a part of the inside wall of a mixer for ground meat. The surface is smooth enough not to interfere with the passage of the mixing blades.

A surface temperature probe (70) is also incorporated, and this too is embedded in said first support (40).

In the case when a mincer is used instead of a mixer, the same configuration of the groups of electrodes shown in Fig. 4 is used, and in this case the surface is positioned on the internal face of a cylinder that is located at the outlet of the mincer. Accordingly, when the meat leaves the mincer its composition is measured. At the end of the cylinder there will be means (for example, a constriction) for increasing the pressure of the meat on the walls of the cylinder so as to improve the contact between the meat and the electrodes.

Another possible embodiment (not shown) for the case of the groups of electrodes of ground meat, is that there is only a first group of electrodes (20, 20') with a first element (20) and a third element (20') for current injection and a second group of electrodes (30, 30') with a second element (30) and a fourth element (30') for detection of voltage drop, with all of them situated adjacent to one another.

In accordance with the method of the invention, the biological medium (10) is submitted to at least two currents $(I_i)$, and more preferably three, of different strength and frequency $(f_i)$, through the first group of electrodes (20, 20'). The voltage drop $(U_i)$ in the biological medium (10) is measured by means of the second group of electrodes (30, 30'). For this, the first element (20) of said first group of electrodes and the second element (30) of said second group of electrodes are positioned in a first zone of said biological medium (10), and the third element (20') of said first group of electrodes and the fourth element (30') of said second group of electrodes are positioned in a second zone of said biological medium (10). Values of electrical impedance $(Z_i)$ are calculated at the applied frequencies $(f_i)$. The values of electrical impedance $(Z_i)$ calculated are adjusted to the following curve:

$$[1] \qquad Z(f) = R_\infty + \frac{R_0 - R_\infty}{1 + (j\,\frac{f}{f_c})^{1-\alpha}}$$

where $R_0$ and $R_\infty$ are the resistances exhibited by the biological medium (10) in direct current and at very high frequency, respectively; $f_c$ is the frequency at which the maximum of the imaginary part is obtained; and $\alpha$ is a parameter connected with the type of tissue of the biological medium (10) being measured.

The relations between the circuit parameters Re and Ri and those of the mathematical model $R_0$ and $R_\infty$ are as follows:

$$[2] \qquad R_e = R_0$$

$$[3] \qquad R_i = \frac{R_0 \cdot R_\infty}{R_0 - R_\infty}$$

where $R_0$ and $R_\infty$ are obtained from the adjustment of equation [1] where the change in impedance with frequency is modeled and presented previously. The relation:

$$[4] \qquad K = \frac{R_\infty}{R_0}$$

gives an estimator of final quality of the meat.

To demonstrate the usefulness of parameter K, we now present a non-limiting example. The processing of ham must avoid the use of two types of legs of pork: PSE legs and DFD legs. PSE stands for pale, soft and exudative. These legs have little capacity for water retention and are of no interest for cured ham (because they give rise to saltier products) or for cooked ham (because of excessive losses of water). The DFD legs (DFD stands for dark, firm and dry) are pieces of meat with high capacity for water retention. These DFD pieces give rise to problems of contamination during the curing process. Parameter K has been shown to be able to detect PSE and DFD meat with a reliability of 90%.

Parameter K can also provide information on capacity for water retention in other meats, such as beef.

For the case of unprocessed meats, and using the groups of electrodes shown in Fig. 2 or in Fig. 3, of the parameters calculated previously, $R_\infty$ is also of interest. This parameter is proportional to the intramuscular fat of the region being measured. If geometric information of the region and information on total weight of the piece are added to the parameter, a high correlation is obtained with the content of intramuscular fat:

$$[5] \qquad I2 = C_x\text{-Weight-q-f}(R_\infty)$$

where I2 is the intramuscular index of the measurement region, $C_x$ is an adjustment constant (it can be zero or non-zero), Weight is the weight of the region or if that cannot be measured the weight of the ham, q is a geometric parameter of the measurement region (for example, the maximum thickness of the leg of pork) and $f(R_\infty)$ is a function of the resistance at infinite frequency, calculated according to the model or measured at a sufficiently high frequency. When Weight is the weight of the region and $C_x$ is adjusted appropriately, I2 can be equal to the intramuscular mass of the region. The function f is usually $1/R_\infty$. It has been demonstrated that by using a value of $R_\infty$ without using the weight and only with coefficients of linear regression, it is possible to estimate the intramuscular fat in *Biceps femoris.*

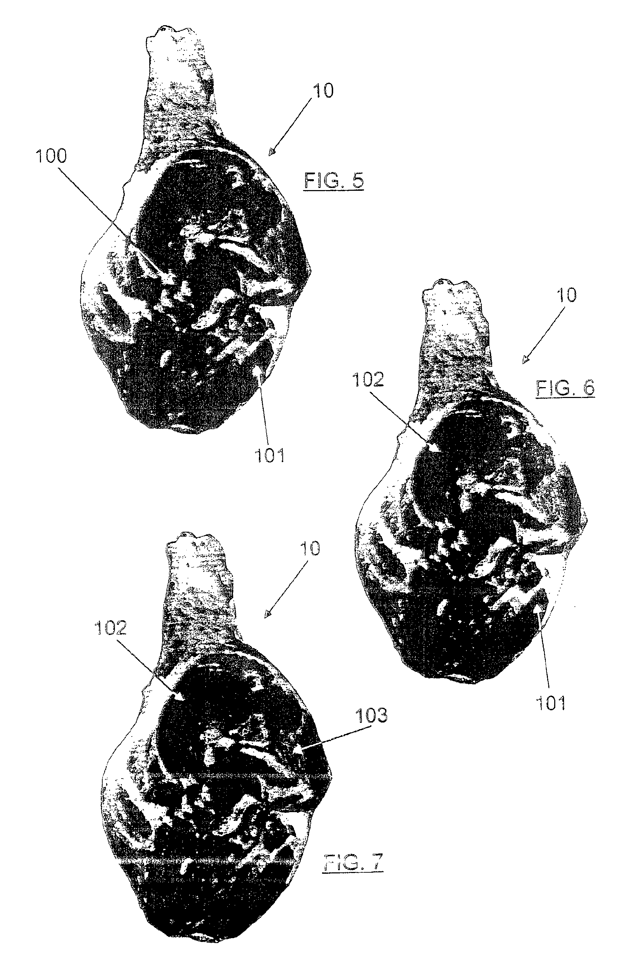

When the groups of electrodes shown in Fig. 3 are used, said first and second zones are different. Examples of first and second measurement zones are shown in Figs. 5, 6 and 7. Each of the drawings shows puncture points for taking measurements of electrical impedance.

In the case of Fig. 5, said first and second zones of the biological medium to be measured (in this case, unprocessed meat) are the *Pectineus* muscle (100) and the *Biceps femoris* muscle (101)

Similarly, in the case of Fig. 6, said zones are the *Semimembranosus* muscle in its distal part (102) and the *Biceps femoris* muscle (101).

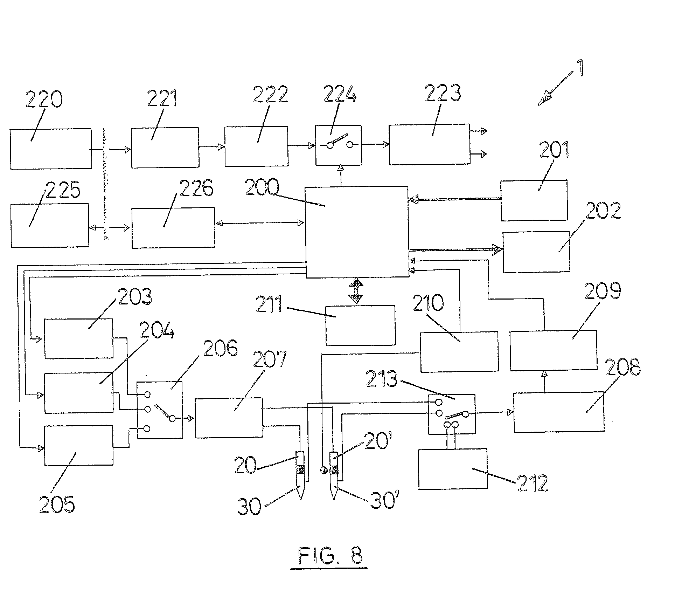

For Fig. 7, said zones are the *Semimembranosus* muscle in its distal part (102) and the *Semimembranosus* muscle in its proximal part (103).

In the case of groups of electrodes such as those shown in Fig. 2, the first and second zones are the same, and can be any one of the puncture points shown in Figs. 5 through 7.

Each position of electrodes in the meat sample, taking into account the internal muscle structures of the piece, can provide information on different zones of the samples. For example, if measurement is carried out with electrode groups as shown in Fig. 3 at points (100) and (101) of Fig. 5, the apparatus gives reliable information on the intramuscular fat of the *Biceps femoris* muscle. This same information is obtained if groups of electrodes as shown in Fig. 2 are used, placed at position (101) in Fig. 5. Similarly, if we use groups of electrodes like those in Fig. 3 positioned at points (102) and (103) as shown in Fig. 7, the apparatus gives reliable information for detecting PSE and DFD meat. The same information is obtained if groups of electrodes are used such as those in Fig. 2 at point (103).

In this way, and applying the method of the invention, the pieces of meat material arriving at the factory can be classified according to quality.

The method of the invention can also be employed for estimating the composition of a sample of ground meat, similarly using the adjustment of the values of the impedance ($Z_i$) measured according to expression [1].

In this case, and using the groups of electrodes shown in Fig. 4, as well as obtaining parameter K according to expression [4] representing the capacity for water retention, measurement of electrical impedance at low frequency can provide information on the fat content FC of ground meat, which has the general expression:

$$[6] \qquad FC = B + D{\cdot}f(R_0)$$

where B and D are constants for adjusting the linear model when comparing the FC measured by an alternative method and the term $f(R_0)$, and $f(R_0)$ is a function of the resistance at low frequency. A concrete case of this expression would be a potential function $FC = 1D{\cdot}(R_0)^a$, where "a" is a constant.

Measurement of electrical impedance at high frequency provides information on the water content of ground meat. Thus, the total water content TWC is obtained from the expression:

$$[7] \qquad TWC = ko_w + k_w{\cdot}G^2{\cdot}f(R_\infty)$$

where $ko_w$ and $k_w$ are constants for adjusting the linear model when comparing the TWC measured by an alternative method with the term $G^2{\cdot}f(R_\infty)$, G is a geometric term of the container where the sample of ground meat is being measured.

A special case of this expression would be a relation that is inversely proportional to TWC:

$$TWC = ko_w + \frac{k_w \cdot G^2}{[R_\infty]}$$

Furthermore, the protein content PC is obtained from the expression:

$$[8] \qquad PC = Weight - TWC - FC - E$$

where Weight is the weight of the sample of ground meat, TWC is the total water content obtained from expression [7], FC is the fat content obtained from expression [6] and E is obtained from standard tables or by calibration.

In this way, and following the method of the invention, it is possible to determine the composition of ground meat as capacity for water retention (parameter K), fat content (FC), total water content (TWC) and protein content (PC).

The method has good capability for measuring these characteristics of composition, as high correlations have been found (of the order of $r^2 = 0.9$) between the stated parameters of composition (K, FC, TWC and PC) obtained using the methods described here and those measured by alternative methods. Therefore, apparatus that effects a frequency sweep on ground meat in a mixer or mincer is a very useful tool for the production process.

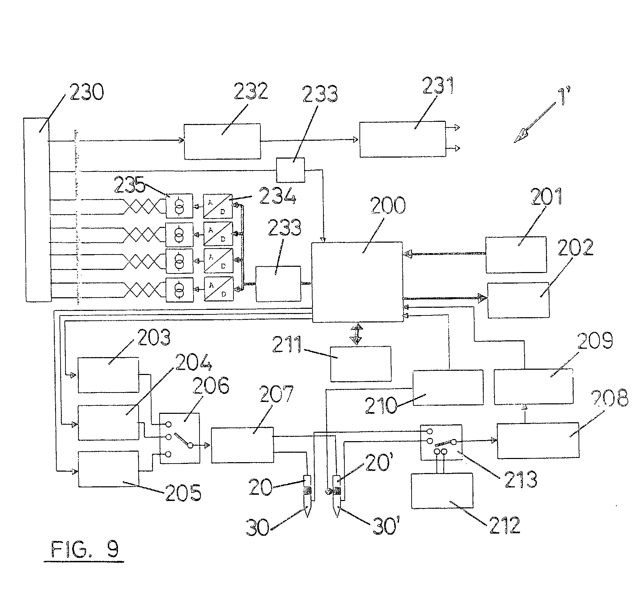

Figs. 8 and 9 show block diagrams of the possible embodiments of the aoparatus (1. 1') of the invention.

Said apparatus (1, 1') for obtaining physical and/or chemical characteristics of a biological medium (10) includes a microprocessor (200), a keyboard (201) and a screen (202). The microprocessor (200) generates a sample that passes through three filters (203, 204, 205) that give rise to three signals of different frequency (typically a low frequency close to $R_0$, a sufficiently high frequency close to $R_\infty$ and a frequency close to $f_c$), which are alternated by a multiplexer (206) for injection by means of a source of current (207) through the first group of electrodes (20, 20'). The voltage drop is measured by means of the second group of electrodes (30, 30') by a circuit that includes a differential amplifier (208); the measurement is compared by a demodulator (209) which enables the microprocessor (200) to calculate the electrical impedance (real part and imaginary part) for each signal. The microprocessor (200) adjusts the three values of impedance. At the same time, the temperature is measured by one of the surface or deep temperature probes (70, 80) using a temperature sensor interface (210). The microprocessor (200) stores said information in a memory (211) and displays it on the screen (202) according to a configuration selected by the user via the keyboard (201). The microprocessor (200) connects a calibration network (212), supplied by the current source (207), when the apparatus is switched on and routinely with a switch (213).

In accordance with the embodiment shown in Fig. 8 the apparatus (1) is connected to the network (220) for charging the battery (222) using a charging circuit (221). This battery is connected to voltage regulators (223) via a switch (224) controlled by the microprocessor (200). The information stored in the memory (211) can be transferred using a computer (225) with special software and via an insulated connection (226), such as optocouplers.

In this case, said apparatus (1) is preferably portable.

In accordance with the embodiment shown in Fig. 9, the apparatus (1') is connected to a control system (230) that controls the operation of the mixer or mincer of ground meat (typically a PLC) for supplying voltage regulators (231) via an insulated converter (232).

In this apparatus (1') the microprocessor (200) controls the execution of measurement according to a signal sent by the control system (230) of the mixer or mincer via an insulated connection (233), typically optocouplers. This signal can indicate passage of the blades over the electrodes to avoid measurement when these are over the electrodes. All the information is transferred via the insulated connection (233) to the control system (230) via various lines, by which a signal is sent that has previously been converted by a group of A/D converters (234) and has been generated in the current sources (235).

**Claims**

1. A method of obtaining physical and/or chemical characteristics of a biological medium (10), comprising submitting said biological medium (10) to at least two currents ($I_i$) of different strength and frequency ($f_i$), via a first group of electrodes, measuring the voltage drop ($U_i$) in said biological medium (10) via a second group of electrodes, locating a first element (20) of said first group of electrodes and a second element (30) of said second group of electrodes in a first zone of said biological medium (10), and locating a third element (20') of said first group of electrodes and a fourth element (30') of said second group of electrodes in a second zone of said biological medium (10), calculating values of electrical impedance ($Z_i$) at the at least two frequencies ($f_i$) applied, **characterised in that** the calculated values of electrical impedance ($Z_i$) are adjusted to the following curve:

$$[1] \qquad Z(f) = R_\infty + \frac{R_0 - R_\infty}{1 + (j\frac{f}{f_c})^{1-\alpha}}$$

where $R_0$ and $R_\infty$ are the resistances exhibited by the biological medium (10) in direct current and at very high frequency, respectively; $f_c$ is the frequency at which the maximum of the imaginary part is obtained; and $\alpha$ is a parameter connected with the type of tissue of the biological medium (10) being measured.

2. A method as claimed in Claim 1, wherein said biological medium (10) is submitted to three currents of different strength and frequency.

3. A method as claimed in Claim 1, wherein the physical and/or chemical characteristics of said unprocessed biological medium (10) are obtained for estimating the quality of said biological medium once processed.

4. A method as claimed in Claim 1, wherein the physical and/or chemical characteristics of said biological medium (10) are obtained for determining its composition.

5. A method as claimed in Claim 1 and 3, wherein said biological medium (10) is unprocessed meat, and the intramuscular index I2 is obtained from the expression:

$$[2] \qquad I2 = C_x \cdot Weight\text{-}q \cdot f(R_\infty)$$

where $C_x$ is an adjustment constant, Weight is the weight of said unprocessed meat, q is a geometric parameter of the meat being measured and $f(R_\infty)$ is a function of the resistance at infinite frequency, with $C_x$, q and $f(R_\infty)$ being obtained by calibration.

6. A method as claimed in Claim 1 and 4, wherein said biological medium (10) is ground meat and the fat content (FC) is obtained from the expression:

$$[6] \qquad FC = B + D \cdot f(R)$$

where B and D are adjustment constants of the linear model when comparing the FC measured by an alternative method and the term $f(R_0)$, and $f(R_0)$ is a function of the resistance at low frequency.

7. A method as claimed in Claim 1 and 4, wherein said biological medium (10) is ground meat and the total water content TWC is obtained from the expression:

$$[7] \qquad TWC = ko_w + k_w \cdot G^2 \cdot f(R_\infty)$$

where $ko_w$ and $k_w$ are constants for adjusting the linear model when comparing the TWC measured by an alternative method with the term $G^2 \cdot f(R_\infty)$, G is a geometric term of the container where the sample of ground meat is being measured.

8. A method as claimed in Claim 1 and 4, wherein said biological medium (10) is ground meat and the protein content PC is obtained from the expression:

$$[8] \qquad PC = Weight \text{-} TWC \text{-} FC \text{-} E$$

where Weight is the weight of the sample of ground meat, TWC is the total water content obtained from expression [7], FC is the fat content obtained according to expression [6] and E is obtained from standard tables or by calibration.

9. A method as claimed in Claim 1 or 2, wherein said first and second zones are the *Pectineus* muscle (100) and the *Biceps femoris* muscle (101).

10. A method as claimed in Claim 1 or 2, wherein said first and second zones are the *Semimembranosus* muscle in its distal part (102) and the *Biceps femoris* muscle (101).

11. A method as claimed in Claim 1 or 2, wherein said first and second zones are the *Semimembranosus* muscle in its distal part (102) and the *Semimembranosus* muscle in its proximal part (103).

12. Apparatus (1, 1') for obtaining physical and/or chemical characteristics of a biological medium (10), that comprises a microprocessor (200), a keyboard (201), a screen (202), means of supplying said microprocessor, a first group of electrodes for injection of current consisting of at least one first. and one third element (20, 20') and a second group of electrodes for measuring the voltage drop, consisting of at least one second and one fourth element (30, 30'), a differential amplifier (208), a demodulator (209) and a calibration network (212), **characterised in that** said apparatus (1, 1') includes at least two filters, which give rise to at least two signals of different frequency that are injected alternately by means of a multiplexer (206) at the first group of electrodes (20, 20').

13. Apparatus as claimed in Claim 12, wherein said apparatus is portable.

14. Apparatus as claimed in Claim 12, wherein it includes means of connection to a control system (230) of a mixer of ground meat, said control system (230) sending a signal to the microprocessor (200) whenever certain blades of the mixer pass over The first and second group of electrodes.

15. Apparatus as claimed in Claim 12, wherein said first group of electrodes and said second group of electrodes are mounted on the same first support (40) and the first element (20) is separated from the second element (30) and the third element (20') is separated from the fourth element (30') by a group of insulating elements (50, 50').

16. Apparatus as claimed in Claim 12, wherein the first element (20) and the second element (30) are mounted on a first support (40) and the third element (20') and the fourth element (30') are mounted on a second support (40').

17. Apparatus as claimed in Claim 12, wherein the first group of electrodes includes at least one fifth element (20") and the second group of electrodes includes at least one sixth element (30"), all the elements (20, 20', 20", 30, 30', 30") being mounted on the same first support (40).

18. Apparatus as claimed in one of the Claims 14-16, wherein the apparatus includes at least one surface temperature probe (70) located on the first support (40).

19. Apparatus as claimed in Claims 14 [lacuna], wherein the apparatus includes a deep temperature probe (80) located on one of the insulating elements (50, 50').

FIG. 1

FIG. 2

FIG. 3

FIG. 4

FIG. 5

FIG. 6

FIG. 7

FIG. 8

EP 1 253 423 A2

FIG. 9

14